# EUROPEAN PATENT SPECIFICATION

(11) **EP 2 903 654 B1**
(45) Date of publication and mention of the grant of the patent: **05.12.2018**
(21) Application number: 13780254.2
(22) Date of filing: 01.10.2013
(51) Int. Cl.: A61L 9/12

(54) **DISPENSING SYSTEM**
AUSGABESYSTEM
SYSTÈME DE DISTRIBUTION

(30) Priority: 02.10.2012 US 201213633791
(43) Date of publication of application: 12.08.2015
(73) Proprietor: S.C. Johnson & Son, Inc., Racine, WI 53403 (US)
(72) Inventor: BELONGIA, David, C., Burlington, WI 53105 (US)
(74) Representative: Ruschke, Hans Edvard
(86) International application number: PCT/US2013/062807
(87) International publication number: WO 2014/055478

(56) References cited:
- WO-A1-01/56623
- WO-A1-2006/074562
- WO-A1-2006/097004
- WO-A1-2012/149114
- WO-A2-2009/002435
- WO-A2-2011/019404
- WO-A2-2011/028259
- US-B1- 7 527 247

## Description

### CROSS REFERENCE TO RELATED APPLICATIONS

Not applicable

### REFERENCE REGARDING FEDERALLY SPONSORED RESEARCH OR DEVELOPMENT

Not applicable

### SEQUENTIAL LISTING

Not applicable

### BACKGROUND OF THE INVENTION

### 1. Field of the Background

The present invention generally relates to a volatile material dispensing system, and more particularly, to a volatile material dispensing system designed to inconspicuously dispense volatile materials in large spaces.

### 2. Description of the Background

Typical volatile material dispensers provide for either the passive or active emission of volatiles into the atmosphere. While passive systems often provide an extended period of volatile material dispersion, they tend not to be particularly effective in distributing volatile materials in large spaces. On the other hand, active volatile material dispensing systems have advantages over passive systems by the inclusion of active dispersion systems such as fans and/or heaters.

For example, one typical active volatile material dispensing system discloses a container having a reservoir filled with a fragrance laden gel. The reservoir includes an open end and a peripheral flange extending therearound. A vapor permeable membrane is secured to the peripheral flange over the open end and an impermeable membrane is removably secured to the permeable membrane. The container is releasably inserted into an electrically heated vapor apparatus. During use, an electrical plug extending from the body is inserted into a conventional wall outlet. Heat from a heating element assists in the volatilization of the fragrance, which is thereafter diffused through the permeable membrane.

In a different active volatile material dispensing system, first and second volatile compositions are disposed within first and second containers. Wicks are used to dispense the volatile compositions in conjunction with heaters (one per wick) associated therewith. Further, a fan may be included in the system for diffusing the volatile compositions.

A third volatile material dispensing system includes a multi-fragrance scent dispenser that includes fragrance-containing gel packs positioned above heater assemblies from which a scent is dispensed. The heaters can be manually controlled. A fan may also be included for diffusing fragrance.
US 7,527,2477 A discloses an apparatus for exposing air to an aromatic substance at a uniform level of aroma for a certain period of time without using gas, fire, or applied electric heat source, comprising an aromatic solution tank on which sits a lid under which is mounted a motor engaged by a micro-switch, wherein disposed on the motor shaft is an impeller generating a guide air flow through a chamber of free space in the tank via an intake housing, inlet and outlet holes in the lid, and wherein rotation of a draw tube delivers a 360 degree sheet of aromatic solution across flowing air, air which is then discharged to the environment.
WO 2009/002435 A1 discloses a device adapted to discharge a volatile material, comprising a display frame having a front face and a rear face and an opening disposed in the rear face and a dispenser disposed within the display frame, wherein the dispenser includes a blister that holds a volatile material and a permeable membrane that extends across an open end of the blister, and wherein the rear face includes an integral foot member connected to an upper portion of the display frame at a hinge, the integral foot member being actuable between first and second states about the hinge.
WO 2012/149114 A1 discloses an apparatus for delivering a compound, comprising a reservoir containing a compound including fragrances and insecticides to be delivered to the environment of use, a compound permeable substrate including a wick which extends from the substrate into the reservoir, wherein a centrifugal fan is operatively associated with the compound permeable substrate to force air over the substrate when the fan is operational, and wherein a cover is disposed over the centrifugal fan in which the cover has at least one aperture on a top surface and provides for a side air passage from the centrifugal fan to the environment.
WO 2006/074562 A1 discloses an apparatus adapted to provide in an atmosphere one of at least three volatile liquids, the apparatus comprising: a source of air current; radially extending from the source of air current a plurality of coplanar air channels corresponding to the number of volatile liquids, placed such that the air current flows through them, each channel leading to a different volatile liquid-emitting member and then into the atmosphere; and located in each channel between the source and the volatile liquid-emitting member, means for blocking the channel and preventing the flow of air therethrough.
WO 2001 / 056623 A1 discloses an apparatus for removing malodor from the air, particularly from confined spaces, utilizing baking soda as the odor removing substance, either alone, or in combination with another odor removing substance which may include activated carbon, the apparatus comprising a detachable cartridge member which contains baking soda and an air moving member that draws air through the detachable cartridge member, wherein a detachable cartridge member with an air moving member is used in one confined space and another cartridge member without the air moving member is used in another compartment of the confined space.
WO 2006/097004 A1 discloses an apparatus adapted to disseminate individually into an atmosphere a plurality of liquids, such as fragrances, the apparatus comprising: a source of forced ventilation; extending radially from the source, a plurality of passages, each having an entrance and an exit for the forced ventilation, the exit leading to the atmosphere; at or near the exit of each passage an evaporative source of volatile liquid; and a movable barrier that surrounds the source, the barrier having one port that is movable into correspondence with a single passage at a time, such that forced ventilation flows down only that passage.
WO 2011 / 019404 A2 discloses a wearable device for dispensing insect repellents, fragrances, and/or other chemicals along the outside of the clothing of a human of the type that is clipped onto a belt or the like and that uses a powered fan to dispense active, comprising a fan rotor arrangement and a rotatable clip structure to render use of the device more comfortable when the user is seated and to provide greater control over the direction of the dispensing.
WO 2011 / 028259 A2 discloses a method of dispensing a volatile active into the surrounding environment, including the steps of providing a fragrance dispenser having a housing, a fan disposed within the housing and having an airflow director, first and second chambers disposed within the housing, and first and second fragrances disposed in the first and second chambers, wherein the first and second chambers include first and second shutters covering first and second outlets, and first and second heaters, respectively, and wherein the method further includes the steps of activating the first heater to volatilize the first fragrance for a first time period, simultaneously activating the fan and the opening the first shutter to dispense the volatilized first fragrance from the first chamber into the surrounding atmosphere, activating the second heater to volatilize the second fragrance for a second period of time, and simultaneously activating the fan and opening the second shutter to dispense the volatilized second fragrance from the second chamber into the surrounding atmosphere.

However, such active volatile material dispensing systems do not adequately address the need to provide volatile material distributed throughout a large space. Typically, such systems are tethered to a wall socket at one end of a room and lack the power sufficient to disperse a volatile active in a room of a size typical in modem houses with open floor plans. Similarly, such devices are also inadequate for the provision of insecticides and/or insect repellents in an outdoor space over a sufficiently large area, such as a patio or deck. The present disclosure contemplates various volatile material dispensing systems that provide for a more effective diffusion of volatiles into the atmosphere of a large room while not needing to be
prominently placed in the room and that have sufficient power to provide effective distribution of volatile materials in an outdoor space.

### SUMMARY OF THE INVENTION

The invention is defined by independent claim 1.

According to one embodiment, a dispensing system for dispensing a material includes a housing for receipt of a dispenser holding a material. A fan is disposed within the housing. Upon activation, the fan draws air into the housing and diffuses the material charged air from the housing with a substantially 360 degree dispersal pattern.

According to another embodiment, a dispensing system for dispensing a material includes a housing having a fan and a dispenser holding a material. A lid is attached to the housing and has a heater. When the lid is in an open state the heater is thermally isolated from the dispenser and unable to heat the dispenser. When the lid is in a closed state the heater is adjacent and in thermal communication with the dispenser.

According to another embodiment, a dispensing system for dispensing a material includes a housing having a heater, a fan and a dispenser holding a material. The dispensing system further includes a lid attached to the housing. When the lid is in a closed state the heater is aligned coaxially with the fan and the dispenser to form a dispensing stack. When the lid is in an open state the heater is not coaxially aligned with the fan and the dispenser.

According to yet another embodiment, a dispensing system for dispensing a volatile active includes a housing, a fan disposed within the housing, and a lid connected to the housing adjacent the top surface. The lid includes a heater. A dispenser is disposed on a top surface of the housing. The dispenser is vertically aligned above the fan. When the lid is in a closed state, the heater is vertically aligned above the dispenser to form a dispensing stack comprising the fan, the dispenser, and the heater. Upon activation, the dispensing stack draws air from a substantially vertical direction above the dispensing stack and exhausts air charged with the heated volatile active with a substantially horizontal 360 degree dispersal pattern.

According to a further embodiment, a dispensing system for dispensing a volatile active includes a housing, a plurality of fans disposed within the housing, and a plurality of heaters disposed within the housing. A heater is associated with each fan. Further, a plurality of dispensers is disposed within the housing and a dispenser is associated with each heater. The dispensing system further includes means for independently controlling the plurality of fans and the plurality of heaters. Each dispenser includes a blister holding a volatile material and a permeable membrane extending across an open end of the blister.

### BRIEF DESCRIPTION OF THE DRAWINGS

Other aspects and advantages of the present invention will become apparent upon reading the following detailed description and upon reference to the drawings in which:
FIG. 1 is an isometric view a dispensing system;
FIG. 2 is a top plan view of the dispensing system of FIG. 1 with a lid in a closed state;
FIG. 3 is a front isometric view of the dispensing system of FIG. 1 with a lid in an open state and a single dispenser provided;
FIG. 3A is a front isometric view of the dispensing system of FIG. 3 with two dispensers shown;
FIG. 4 is a partial front isometric view of the dispensing system of FIG. 3 with the lid and dispenser removed;
FIG. 5 is a front isometric view of the dispenser of FIG. 3;
FIG. 6 is a rear isometric view of the dispenser of FIG. 3;
FIG. 7 is a cross-sectional view of the dispenser of FIG. 5 along the lines 7-7 of FIG. 5 in a first condition;
FIG. 8 is a cross-sectional view of the dispenser of FIG. 5, similar to FIG. 7, showing the dispenser in a second condition;
FIG. 9 is a front isometric view of the dispensing system of FIG. 4 with a top surface of the housing removed;
FIG. 10 is a front isometric view of the dispensing system of FIG. 9 with an impeller removed from one side;
FIG. 11 is an exploded isometric view of the dispenser of FIG. 1;
FIG. 12 is a top plan view of the dispensing system of FIG. 1 illustrating its operation according to one embodiment;
FIG. 13 is a front elevational view of the dispensing system of FIG. 12;
FIG. 14 is a schematic view of a dispensing system according to another embodiment;
FIG. 15 is a schematic view of a dispensing system according to a further embodiment; and
FIG. 16 is a schematic view of a dispensing system according to a still further embodiment.

### DETAILED DESCRIPTION OF THE INVENTION

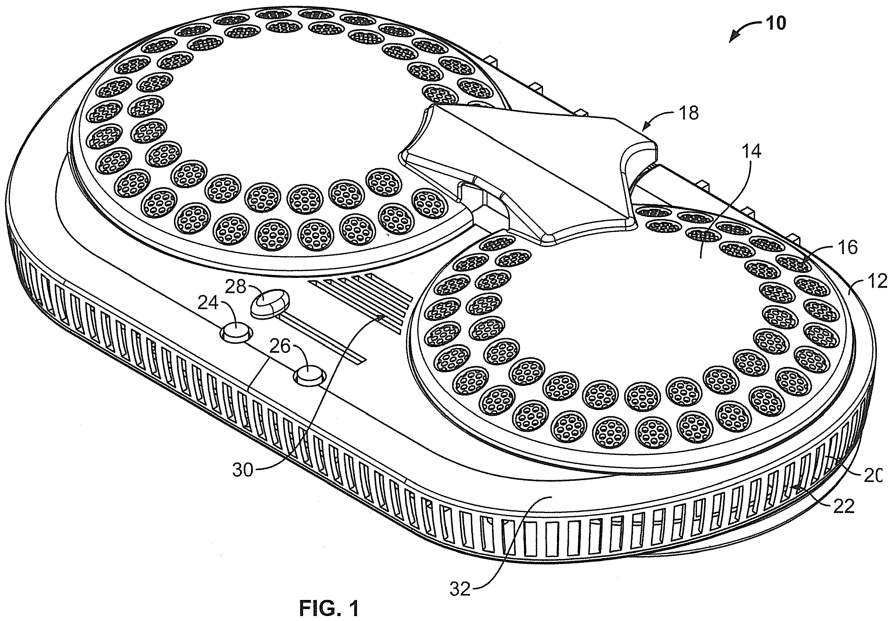

Referring to FIGS. 1-4 and 9-13, a volatile material dispensing system 10 is illustrated according to one embodiment. The dispensing system 10 includes a housing 12 and a lid 14. The lid 14 includes vents 16 therein and may be attached to the housing 12 by way of a hinge 18 or similar means. The housing 12 includes a sidewall 20 with vents 22 disposed therein. The vents 22 substantially circumscribe the periphery of the housing 12. A power switch 24, a reset switch 26, a fan speed control 28, and speaker apertures 30 are disposed on a top surface 32 of the housing 12. In other embodiments, one or more of the aforementioned switches, controls, or apertures may be removed or consolidated, e.g., the dispensing system 10 could be provided without a speaker and speaker apertures. The power switch 24 and reset switch 26 may be LED-containing push button-type switches as are known in the art. The fan speed control 28 is illustrated as a sliding switch and may be a rheostat or the like, or any other type of control mechanism that allows for variable speed control of an electric motor.

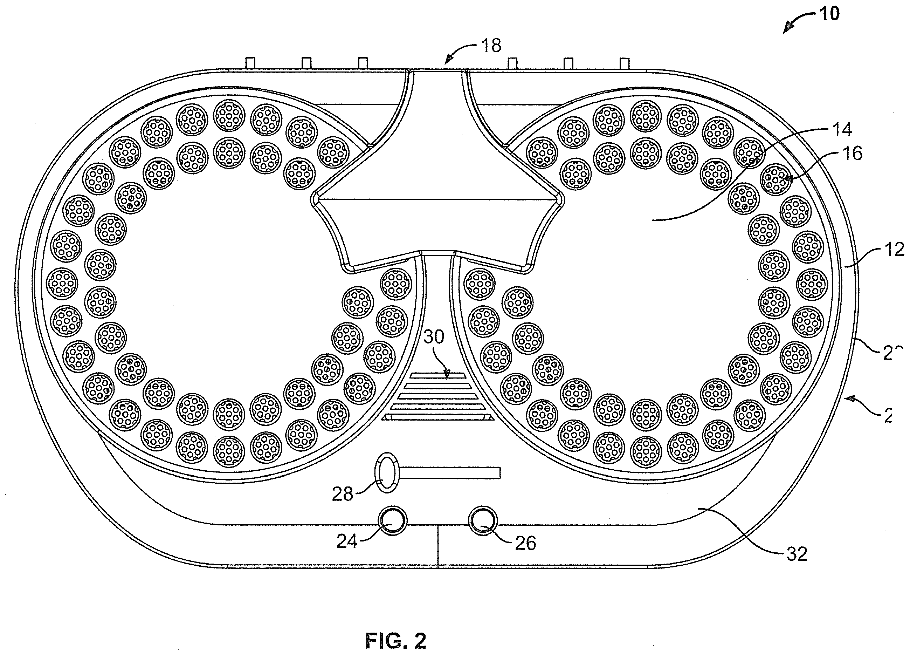

The dispensing system 10 may have a generally flat, oval or oblong circular shape when viewed from above or below, as seen in FIGS. 1 and 2, but may also be circular, rectangular, elliptical, triangular, or any other shape. The housing 12 and lid 14 may be constructed from any suitable material, such as a plastic, a polymer, a metal, glass, wood, stone, other natural elements, or combinations thereof. Additionally, the materials may include combinations of manufactured, natural, and recycled or reclaimed materials.

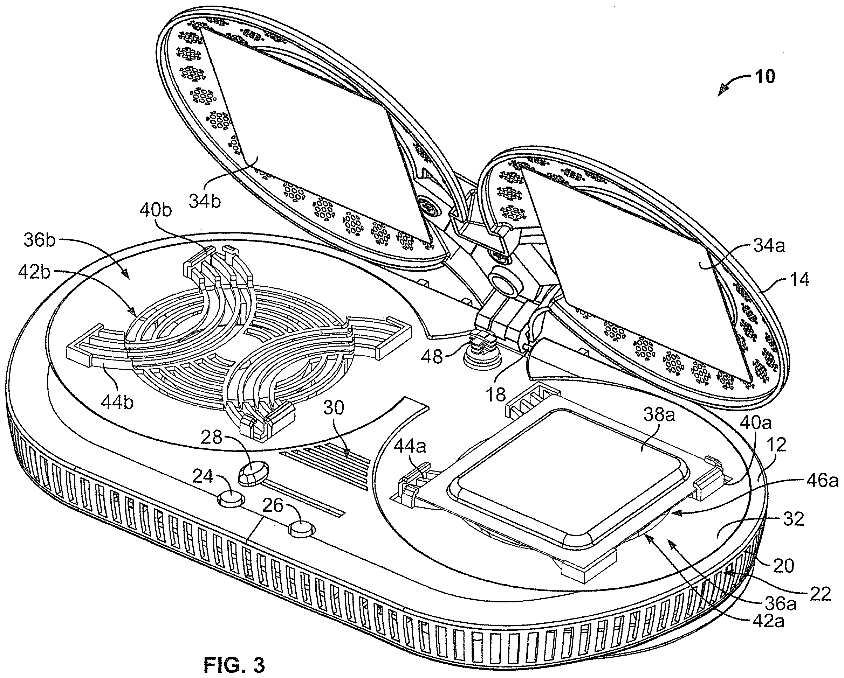

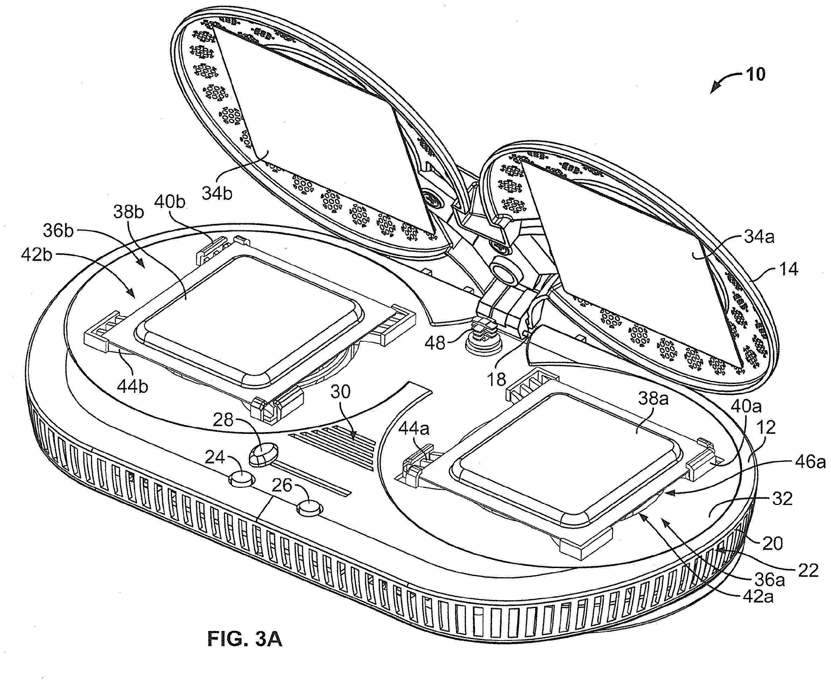

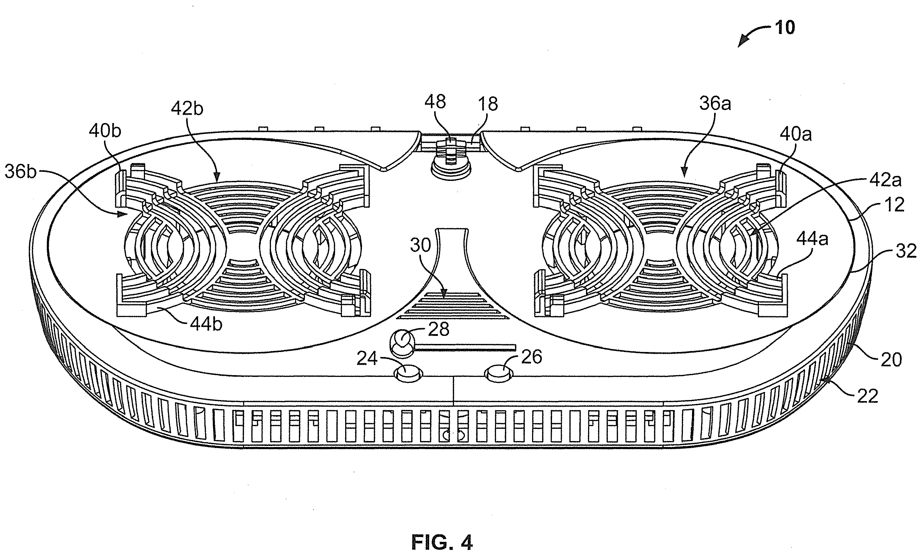

FIG. 3 depicts the dispensing system 10 with the lid 14 in an open state to reveal two heaters 34a, 34b. Further, two seats 36a, 36b are provided for the receipt of two dispensers 38a, 38b. In the embodiment shown in FIG. 3, only one dispenser 38a is shown for purposes of clarity (see FIG. 3A, where both dispensers 38a, 38b are shown). However, it is envisioned that any number of heaters and dispensers may be employed in the dispensing systems of the present disclosure, e.g., one heater and dispenser, three heaters and dispensers, four heaters and dispensers, etc. Further, in the present embodiment shown, the right side of the dispensing system is substantially the same as the left side, such that the description of a feature on the right that is duplicated on the left equally applies thereto. In FIGS. 3 and 3A, the dispensers 38a, 38b are retained within the seats 36a, 36b on the top surface 32 of the housing 12 by retention means 40a, 40b, such as a snap fit or a friction fit mechanism, or any other suitable means known in the art. The dispensers 38a, 38b are vertically aligned above vents 42a, 42b through the top surface 32 of the housing 12 (see also FIG. 4). Furthermore, the dispensers 38a, 38b are spaced from the top surface 32 of the housing 12 by vent spacers 44a, 44b that create passages 46a, 46b for air to pass between the dispenser and the top surface of the housing and through the vents 42a, 42b.

When in a closed state, the lid 14 is secured in place by lid retention means 48, such as a snap fit or a friction fit mechanism, or any other suitable means known in the art. It is envisioned that the dispensing system 10 may be rendered operable in the closed state by completion of a circuit to enable power to be routed to the power switch 24. Further, when in the closed state, the heaters 34a, 34b are adjacent the dispensers 38a, 38b such that they are in thermal communication and heat generated by the heaters passes to the dispensers to heat a volatile active-containing material to increase its rate of volatilization. It follows that when the lid 14 is in an open state, the heaters 34a, 34b are thermally isolated from the dispensers 38a, 38b such that the heater cannot effectively heat the volatile active-containing material held within the dispensers.

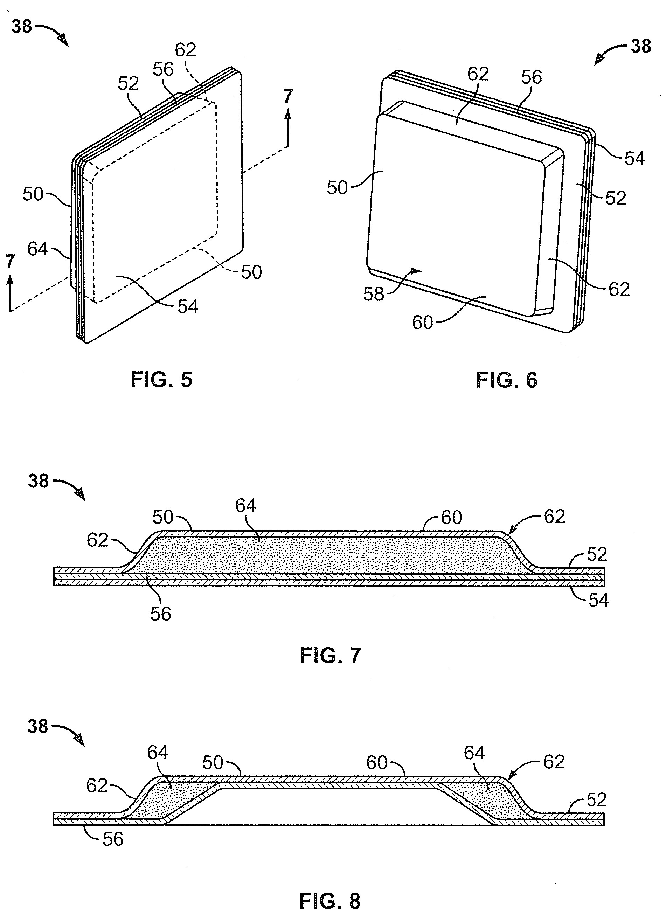

The dispenser 38 is further illustrated in FIGS. 5-8. With reference to FIGS. 5 and 6, the dispenser 38 or cartridge comprises a blister 50, a peripheral flange 52, and an impermeable laminate 54 releasably adhered to the blister 50 and the flange 52. The blister 50 includes a nonporous permeable membrane 56 and a cup-shaped structure 58 or reservoir. The cup-shaped structure 58 includes a bottom wall 60 and four side walls 62 that in conjunction with the permeable membrane 56 act as a sealed reservoir to contain a volatile material 64 (shown in FIGS. 7 and 8). Illustratively, the cup-shaped structure 58 and the permeable membrane 56 are formed from clear and/or translucent materials, thereby allowing the volatile material 64 to be visible therethrough. The peripheral flange 54 is planar and is coupled to and extends outwardly from top edges of the cup-shaped structure 58. In one embodiment, the peripheral flange 54 extends outwardly from upper edges of the side walls 62 and is integrally formed therewith. The present dispenser 38 and the volatile material 64 are similar to those described in U.S. Patent Nos. 7,213,770, 7,523,577, and 7,665,238.

FIG. 7 illustrates the dispenser 38 in a first condition. The dispenser 38 is completely or substantially full in the first condition, i.e., little or no volatile material 64 has diffused through the permeable membrane 56 because the impermeable laminate 54 has not been removed from the blister 50. There is substantially no diffusion of the volatile material 64 when the dispenser 38 is filled and the impermeable laminate 54 covers the permeable membrane 56. Illustratively, the impermeable laminate 54 is removed from the blister 50 by a user grasping an end of the impermeable laminate 54 and peeling it off the blister 50. A tab, extension, or other means for grasping may be included as an extension of the impermeable laminate 54 to aid in removal of same. The extension may be at the corners, ends, and/or on the surface of the impermeable laminate 54.

Following removal of the impermeable laminate 54, the dispenser 38 begins to transition from a full or first condition (FIG. 7) to an empty or second condition (FIG. 8). There may be a small amount of the volatile material 64 that remains in the blister 50 and the dispenser 38 will still be considered to have reached the second condition. As the volatile material 64 diffuses through the permeable membrane 56, the permeable membrane 56 slowly collapses upon the bottom wall 60. With reference to FIG. 8, following diffusion of the volatile material 64 across the permeable membrane 56 there is less volatile material contained within the dispenser 38. Substantially no new air enters the dispenser 38 subsequent to diffusion of the volatile material 64. The result of this is a pressure gradient across the permeable membrane 56, with a higher pressure existing in the ambient air than the pressure in the dispenser 38. The pressure gradient causes the ambient air to exert a net positive pressure upon the dispenser 38, which presses the permeable membrane 56 against the remaining volatile material 64 and ultimately the bottom wall 60.

The volatile material 64 may include a fragrance, an insecticide, a deodorizer, a fungicide, a bacteriocide, a sanitizer, a pet barrier, or other active volatile or other compound disposed within a carrier liquid (for example, an oil-based and/or water-based carrier), a deodorizing liquid, or the like. Examples of possible insecticides include metafluthrin and transfluthrin, among others.

Additional examples of the volatile material 64 include OUST™, an air and carpet sanitizer for household, commercial, and institutional use, or GLADE®, a household deodorant, both sold by S. C. Johnson and Son, Inc., of Racine, Wisconsin. The volatile material 64 may also comprise other actives, such as sanitizers, air and/or fabric fresheners, cleaners, odor eliminators, mold or mildew inhibitors, insect repellents, and the like, or others that have aromatherapeutic properties. The volatile material 64 alternatively comprises any fluid known to those skilled in the art that can be dispensed from a container, such as those suitable for dispersal in the form of particles or droplets suspended within a gas and/or propelled by means of a propellant. The dispensing system 10 is therefore adapted to dispense any number of different fluid or product formulations.

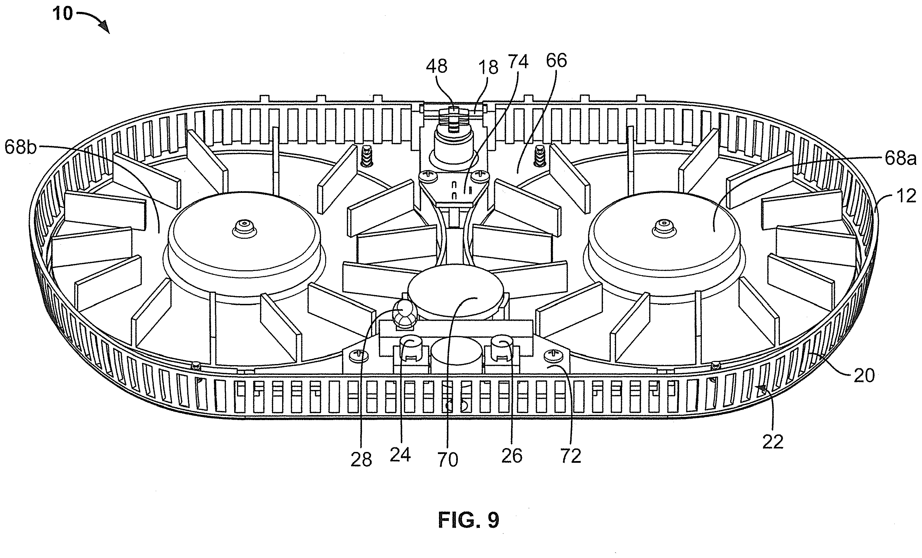

Turning now to FIG. 9, the dispensing system 10 is illustrated with the lid 14 and top surface 32 removed to reveal a bottom surface 66 and two impellers or fans 68a, 68b provided adjacent the bottom surface. Further, an optional speaker 70 is shown as well as a control board 72. FIG. 9 also depicts a power connector 74 where an AC power cord (not shown) may be connected to the dispensing system 10. In another embodiment, batteries or other power sources may be used to power one or more of a heater and a fan of the dispensing systems disclosed herein.

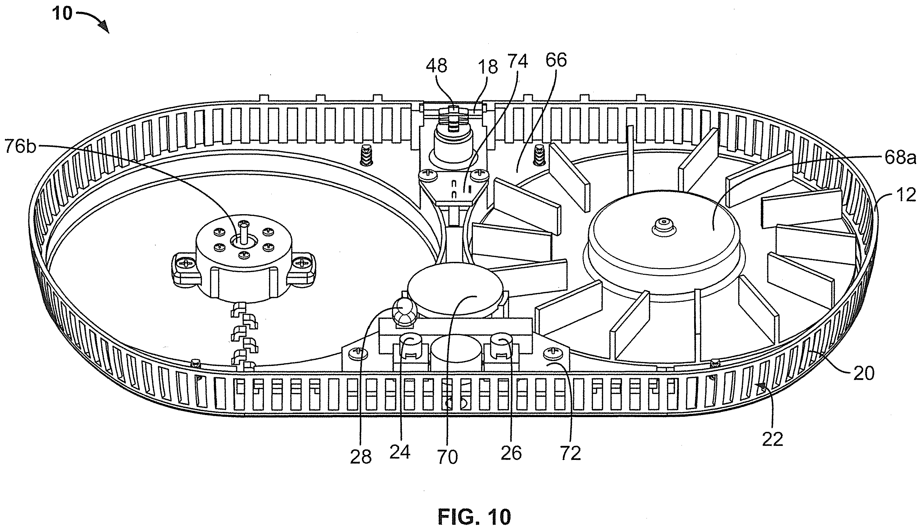

With reference to FIG. 10, the dispensing system 10 is shown with the second fan 68b removed to reveal the placement of a fan motor 76b (motor 76a not shown). Dispensing systems of the present disclosure may employ either DC motors or AC motors, as desired. In the present system, the motors 76a, 76b are DC motors.

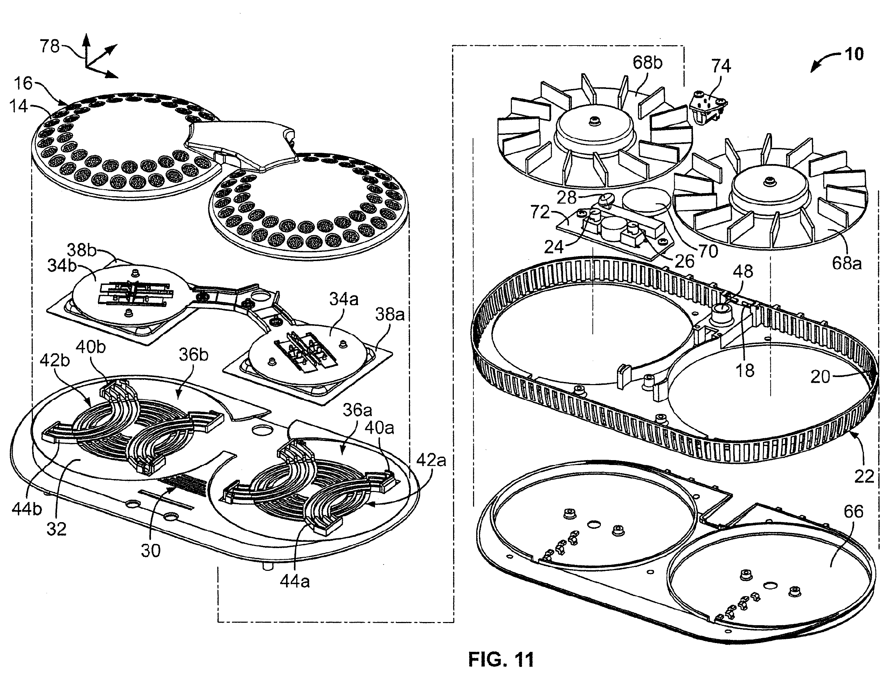

FIG. 11 illustrates an exploded view of the dispensing system 10. Here, the relative assembly of the dispensing system 10 is illustrative of the coaxial stacking of functional components to form dispensing stacks along an axis 78, which in the embodiment shown is generally parallel to vertical. The general structure enables the dispensing system 10 to draw air from one direction, for example one substantially parallel to the vertical axis, and distribute volatile material charged air in a direction substantially perpendicular to the direction of air intake. Beginning from the bottom, the bottom surface 66 and side wall 20 form a foundation for the fans 68a, 68b, a speaker 70, a control board 72 (with various controls 24, 26, and 28), and a power connector 74. The top surface 32 is then placed thereon to complete the housing 12. Now beginning from the top, the lid 14 serves as the foundation for the heaters 34a, 34b, which nest within a concave portion (not seen) of the lid. FIG. 11 further demonstrates the relative orientation of the heaters 34a, 34b with respect to the dispensers 38a, 38b, respectively, in order to establish thermal communication therebetween. Therefore, the relative proximity of the heaters 34a, 34b with the dispensers 38a, 38b during use of the dispensing system 10 is seen.

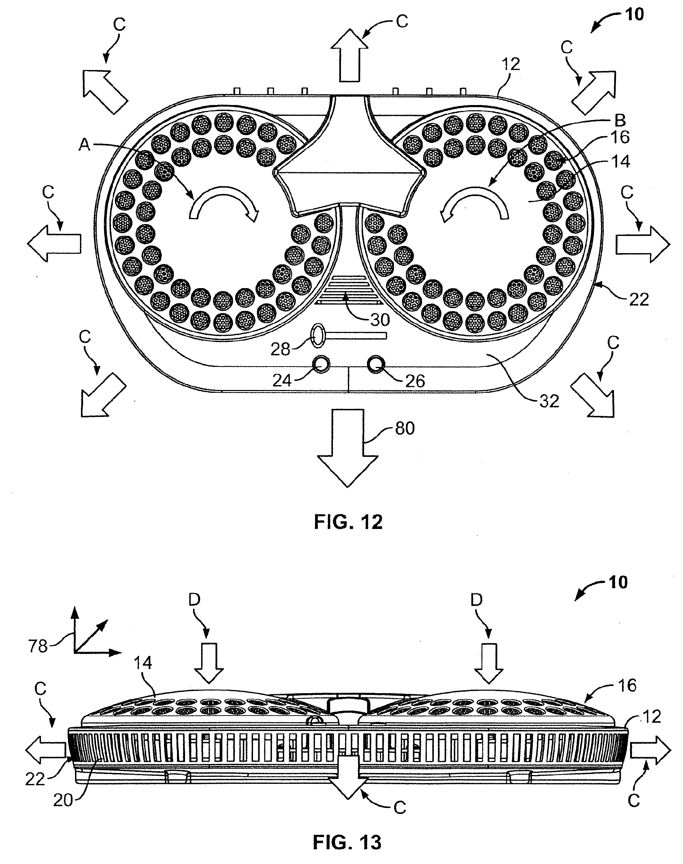

Turning to FIGS. 12 and 13, an air intake and distribution pattern of a dispensing system 10 according to one embodiment is illustrated. As previously described, the dispensing system 10 includes two coplanar fans 68a, 68b. According to the present embodiment, each fan 68a, 68b has a direction of orientation that is counter-rotational with respect to the other, as shown by arrows A and B. In use, the fans 68a, 68b create a dispersal pattern of air, which may be described as a dispersion plane, through the vents 22 in the side wall 20 of the housing 12 that spans substantially 360 degrees around the dispensing system 10 as illustrated by arrows C. Further, the counter-rotation of the fans 68a, 68b creates a primary distribution vector 80 where the exhausts of the two fans are additive. In this way, the primary distribution vector 80 has a larger magnitude or volume of air in one direction relative to the average volume of air distributed in other directions of the substantially 360 degree dispersal pattern. For example, if the relative volume of air per second measured anywhere along the substantially 360 degree dispersal pattern outside of the primary distribution vector 80 is represented by the variable X, then the relative volume of air per second measured at the primary distribution vector 80 may have a value of > X. In one embodiment, the relative volume of air per second measured at the primary distribution vector 80 is 1.5X, or 2X, or 3X, or less or more. Similarly, the primary distribution vector 80 will also have a greater output per unit of time of the material or volatile dispensed or diffused from the dispenser(s) 38 relative to a base value of material or volatile output measured per unit of time anywhere along the 360 degree dispersal pattern outside of the primary distribution vector. For example, in one embodiment the primary distribution vector 80 may be at 120%, or 150%, or 200%, or 500%, or any value in between or less or more relative to the base value of material or volatile output measured per unit time anywhere along other portions of the 360 degree dispersal pattern. In principle, a user may choose to place the dispensing system 10 with the primary distribution vector 80 facing a portion of a room or a space where a greater amount of the material or volatile active is desired. In another embodiment, the dispensing system 10 may have a plurality of fans having the same direction of rotation or a single fan, such that there would not be a primary distribution vector 80.

Further, air intake into the dispensing system 10 occurs through the vents 16 in the lid 14, as illustrated in FIG. 13. The direction of air intake into the dispensing system 10 is indicated by arrows D, i.e., substantially parallel to axis 78, which may be otherwise referred to as an intake axis. Thus, when activated, the fans 68a, 68b (not shown) draw air into the housing 12 of the dispensing system 10 from a first direction, which in the present embodiment is a substantially vertical direction represented by arrows D, and exhaust air charged with the volatile active 64 heated by the heaters 34a, 34b from the housing in a second direction, which in the present embodiment is a substantially horizontal 360 degree dispersal pattern illustrated by the arrows C. Preferably, the axis 78 or intake axis is substantially perpendicular to the dispersion plane. In another embodiment, the air is drawn into the housing from an angle equal to or substantially less than about 45 degrees from the axis 78. In another embodiment, the angle between the air intake vector (D) and the air distribution vector (C) ranges from about 45 degrees to about 1 35 degrees inclusive.

Due to this air flow pattern, the dispensing systems of the present disclosure may be placed with the fan 68a, 68b in a substantially horizontal orientation (primary orientation), for example, on the floor beneath furniture, such as a couch or a bed, or placed in a narrow space in a vertical orientation (secondary orientation), such as between a bookcase and a wall. In this way, the dispensing systems of the present disclosure may be used in an inconspicuous manner to provide a large volume of volatile active-charged air in a desired environment, such as a great room of a house.

In operation, a user places at least one dispenser 38 in the dispensing system 10 before activation. In the present embodiment, it is preferred to place two dispensers 38a, 38b into the dispensing system 10. To prepare the dispensers 38a, 38b for use, the impermeable laminates 54 are removed to expose the permeable membranes 56. The dispensers 38a, 38b are then placed within the retention means 40a, 40b with the permeable membranes 56 facing the vents 42a, 42b and vent spacers 44a, 44b. The user may then close the lid 14 and activate the dispensing system 10 by depressing the power switch 24, which provides power to the heaters 34a and 34b and to the fans 68a and 68b. The fan speed control 28 enables the user to choose between various volumetric dispensing settings. A low setting allows a minimal volume of volatile material charged air to be dispensed, which may be desirable for a maintenance dose of volatile material in a room. Alternatively, a high setting may be selected when a larger volume of volatile material charged air is desired and/or a larger room is being treated.

In another example of operation, the dispensing system 10 may be used without the heaters 34a, 34b or fans 68a, 68b. In such a state, the dispensing system 10 has a baseline volatile material release rate. It is contemplated that additive activation may be employed by a user to increase the rate of volatile material release in an incremental manner. For example, a user may choose to operate only one heater 34a without the other heater 34b and without either fan 68a, 68b. Further, the user may wish to add a second heater 34b or a single fan 68a or 68b, which may or may not be paired with the heater 34a in use. As well, both heaters 34a, 34b may be used without either fan 68a, 68b, or a single fan may be used with both heaters, or both heaters and both fans may be used. In another embodiment, the power switch 24 may also control the temperature of the heaters 34a, 34b.

In a further example of operation, a user may prepare the dispensing system 10 as previously described and place the system with an orientation that aligns the intake axis to be substantially parallel with vertical, thus orienting the dispersion plane to be substantially parallel with horizontal, such as for example on a floor of a room or patio. In this example, the intake axis is substantially perpendicular to the dispersion plane. Alternatively, the same dispensing system may be placed against an upright structure such that when in use, the intake axis is substantially horizontal and the dispersion plane is substantially parallel with vertical.

In a further embodiment, a plurality of preprogrammed sequences of activation and deactivation of the heaters 34a, 34b and/or fans 68a, 68b may be chosen via power switch 24. In one example, a sequence may activate both heaters for a period of time and then activate the fans once the heaters have heated the volatile material to a predetermined temperature to achieve a particular diffusion rate of the volatile material. For example, in one sequence cycle, the heaters heat the volatile material to a predetermined temperature. Upon reaching that temperature, the fans are activated and the heaters may remain activated or may be deactivated to save energy while the fans run. The fans may run for a predetermined time, or alternatively, may run until the temperature of the volatile material drops to a predetermined temperature which triggers the deactivation of the fans. Upon deactivation of the fans, the cycle may begin again with the heaters being activated. The predetermined sequences may be varied by the inclusion or exclusion of heaters and fans, durations of activation time, and activation/deactivation conditions, such as time and/or heat.

Upon first use of a new dispenser(s) 38, the user may depress/select the reset switch to activate a countdown sequence. The countdown sequence may be for any suitable period of time, such as, one or more hours, one day, two days, several days, 1 week, 2 weeks, 3 weeks, a month, several weeks or months, 10-14 days, 20-28 days, or for shorter or longer, or any duration of time in between. Once the countdown sequence reaches an end a signal is emitted to indicate the need to replace the dispenser(s) 38. In one optional embodiment, the signal may be a sound, such as a ring tone or any other sound, which is played through the optional speaker 70 to indicate to the user that the dispenser(s) 38 should be replaced. Alternatively or in addition, the signal may be light-based or vibratory and the like.

It is contemplated that any type of preprogrammed timing sequence may be used in connection with the dispensing system 10. For example, in one embodiment the dispensing system 10 runs the heaters 34a, 34b and fans 68a, 68b continuously upon activation by the user, in which the user selects the fan speed as noted above. Alternatively, means may be provided to adjust the heater intensity. In a different embodiment, the device runs continuously according to a pre-programmed sequence in which the heaters 34a, 34b and fans 68a, 68b for both of the dispensers 38 are continuously run or powered down according to a timing sequence. For example, the dispensing system 10 could be run for a specified period of time, e.g., one or more minutes, hours, days, weeks, or months, and off for another specified period of time that could be the same or different than the on period of time, e.g., one or more minutes, hours, days, weeks, or months. In another embodiment, the device runs continuously, but intermittently powers the heaters 34a, 34b and fans 68a, 68b to one or more of the dispensers according to a random sequence. In yet another embodiment, a pre-programmed or random sequence such as those described above is applied to each dispenser 38 so that one dispenser operates according to one sequence and one or more other dispensers operate according to a different sequence, e.g., the heater 34a and fan 68a are on for a specific period of time and subsequent to the lapsing of the specific period of time the heater 34b and the fan 68b are run in connection with a different dispenser 38.

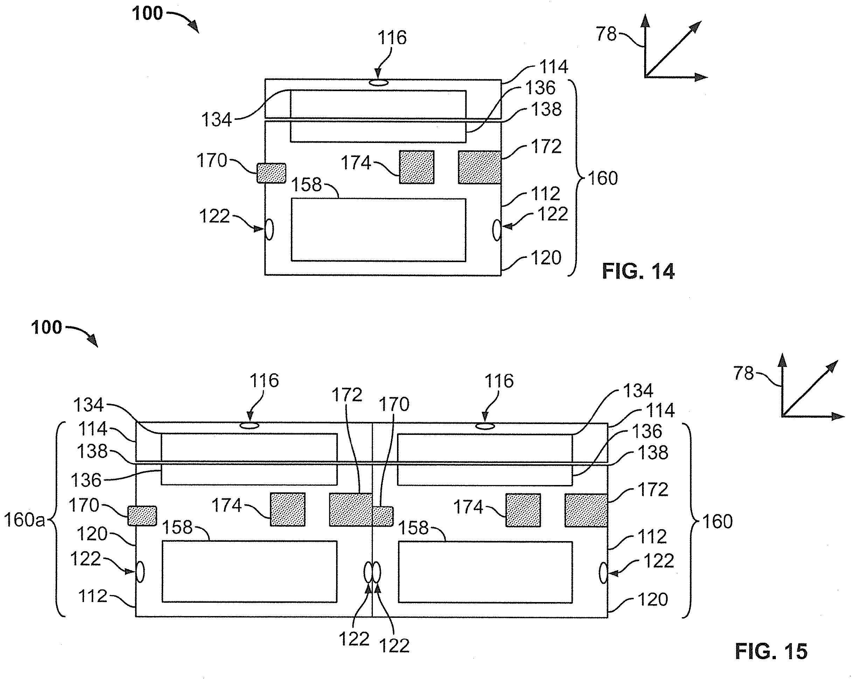

In another embodiment shown in FIG. 14, a dispensing system 100 includes a housing 112 and a lid 114 with a vent 116 therein. The lid 114 comprises a heater or other actuation mechanism 134, which aligns atop a dispenser or other volatile containing reservoir or volatile laden structure 136 disposed on a top surface 138 of the housing 112. The reservoir or structure 136 is likewise aligned above a fan or other diffusion or dispersal mechanism 158 to create a dispensing stack 160. Upon activation the dispensing stack 160 draws air from a first direction, e.g., in a direction substantially parallel to the axis 78, above the dispensing stack and exhausts air charged with the material or volatile active in a second direction, e.g., in a 360 degree dispersal plane substantially perpendicular to the axis 78, through side vents 122 in the housing 112. The dispensing stack 160 further includes a control system 170 for controlling the actuation mechanism 134 and/or diffusion or dispersal mechanism 158, e.g., the control system 170 can turn the heater and/or fan on and off and control the amount of heat and/or speed of the fan. In one embodiment, the dispensing system 100 is identical to the dispensing system 10.

The control system 170 may be manual, automatic, timer, or sensor based as is known in the art. For example, the control system 170 may include a secondary activation means 172 to effectuate diffusion of a material from the dispenser/reservoir/structure 136 besides through a pre-programmed or random timing sequence, as described above. It is contemplated that the secondary activation means 172 may be a pushbutton or instant activation switch that a user can actuate to provide for the operation of one or more of the actuation mechanism(s) 134 and/or diffusion or dispersal mechanism(s) 158 or a boost in the operation thereof, e.g., heaters and/or fans may be run to output more heat or diffused air, respectively. In lieu of the instant activation switch, or in conjunction with such a switch, the secondary activation means 172 may comprise or include a sensor for activation of one or more of the actuation mechanism(s) 134 and/or diffusion or dispersal mechanism(s) 158. In these embodiments, activation may be responsive to sensory input from one or more sensors that detect one or more environmental conditions and/or objects or people, of which a non-exclusive list of sensors includes light sensing elements, such as photodetectors, photodiode light detectors, photoresistors, photodiodes, or phototransistors; passive infrared sensors; motion sensors; acoustic sensors; humidity sensors; temperature sensors; pressure sensors; vibration sensors; accelerometers; and chemical sensors. It is further contemplated that various power supplies 174, such as batteries, rechargeable batteries, solar cells, power cords, and the like may be included.

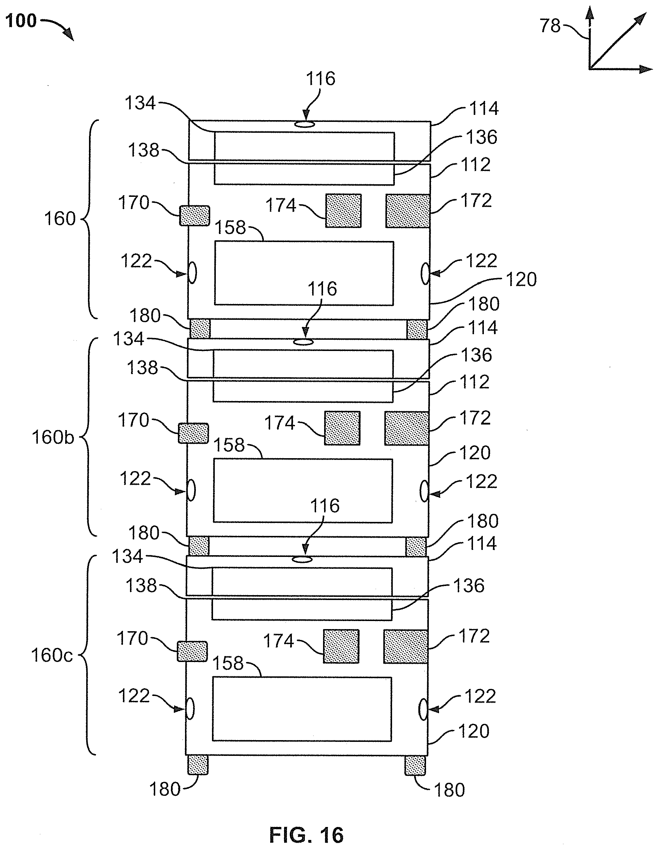

In a further embodiment of a dispensing system shown in FIG. 15, the dispensing stack 160 is a modular component that can be added to another dispensing stack 160a to enable greater distribution of volatile active than from a single dispensing stack. It is envisioned that any number of dispensing stacks may be interconnected to form a variably sized dispensing system 100. While the dispensing stacks 160, 160a of FIG. 15 are illustrated in a horizontal configuration, it is envisioned that they may also be configured in a vertical configuration as shown in FIG. 16. Here, feet 180 on the bottom of each dispensing stack 160 serve as spacers when stacked vertically to provide adequate room for air intake in middle 160b and bottom 160c placed dispensing stacks.

The following alternative embodiments, while described in the context of FIGS. 14-16, are equally applicable to the dispensing system 10 described in FIGS. 1-4 and 9-13, which could be similarly modified to include the below noted structure. To begin with, the dispenser(s) 38, 136 may comprise a container or housing structure with an open end or one or more openings, a container or reservoir with a vapor or liquid permeable membrane extending across one or more openings, a container or reservoir having one or more wicks extending therefrom, etc. In such embodiments, the actuation mechanism 134 may include a heater or heating element that heats one or more of a container or reservoir, one or more wicks extending from a container or reservoir, or an area adjacent one or more wicks. Further, in other embodiments, the actuation mechanism 134 could include one or more of a piezoelectric element or plate adjacent a wick or a mechanism that opens a window or otherwise removes an obstruction from one or more of an open end or openings in a container or reservoir. Still further, the diffusion or dispersal mechanism 158 may include one or more fans, diffusers, nebulizers, or mechanisms to actively force air through the dispensing system 10, 100.

In a different embodiment, the actuation mechanism 134 may include other conventional electronic dispensing means that spray fluid from an aerosol container, whether metered or non-metered, and pump-type sprayers, whether pre-compression or non pre-compression pump-type sprayers, that constitute the dispenser 38, 136. Conventional actuation mechanisms 134 may include, but are not limited to, mechanically driven means, such as armatures, levers, linkages, cams, etc., that depress, tilt, or otherwise activate a valve stem or pump of a container by direct interaction with the valve stem or pump, through indirect communication with the valve stem or pump, and/or through physical interaction with the container, i.e., lifting, pushing, tilting, lowering, or otherwise deflecting the container to effect the depression or tilting of the valve stem or pump. It is also contemplated that solenoid actuators, bi-metallic actuators, muscle wire actuators, piezo actuators, or any other means may be utilized to effect spraying of an aerosol or pump type container. In this embodiment, any of the aforementioned diffusion or dispersal mechanisms 158 may be utilized, e.g., one or more fans.

Further, it is also contemplated that other embodiments may utilize different dispensers 38, 136 and actuation mechanisms 134. For example, the actuation mechanism 134 may comprise a nebulizer or venturi sprayer used in conjunction with any of the aforementioned dispensers 38, 136. Still further, any of these systems 10, 100 may utilize a material or fluid provided within a container or reservoir 38, 136 that is pressurized or non-pressurized. In yet another embodiment, the reservoir 38, 136 may comprise a volatile laden structure such as one or more candles, fragrance blocks, wax melts, or products, whether solid or gel, that allow for the diffusion of an active or volatile through the melting or heating thereof. Similar to the previous embodiment, any of the aforementioned diffusion or dispersal mechanisms 158 may be utilized, e.g., one or more fans.

The dispensing systems contemplated herein may be of any size. For example, the dispensing systems 10, 100 may have a length of about 1 to about 2 inches, or about 2-3 to about 4-5 inches, or about 4-5 to about 6-7 inches, or about 6-7 to about 8-10 inches, or about 8-10 to about 12-16 inches, or more or less. The dispensing systems 10, 100 may also have a width of about 1 to about 2 inches, or about 2-3 to about 4-5 inches, or about 4-5 to about 6-7 inches, or about 6-7 to about 8-10 inches, or about 8-10 to about 12-16 inches, or more or less. The dispensing systems 10, 100 may also have a height (oriented along the intake axis) of about 1/2 to about 1 inch, or about 1 to about 2 inches, or about 2-3 to about 4-5 inches, or about 4-5 to about 6-7 inches, or about 6-7 to about 8-10 inches, or about 8-10 to about 12-16 inches, or more or less. With reference to the dispensing systems depicted in FIGS. 15 and 16, wherein the dispensing systems may be modular and unlimited in terms of the numbers of dispensing stacks 160 incorporated, the individual dispensing stacks may be sized as described above and the size of the dispensing units based on the number of dispensing stacks incorporated in the dispensing system.

Moreover, the dispensing systems and/or dispensing stacks of the present disclosure may be sized according to desired use. For example, a dispensing system for personal use, such as may be used to dispense an insecticide or insect repellent, may be sized to fit into a user's pocket, such as about 4-5 inches long, 1/2 inch high, and about 4-5 inches wide. As another example, when the dispensing system is to be deployed in a book shelf, for example as a book end, the dispensing system may be sized according to the shelf height and/or to approximate the size of a book, for example, 10 inches long, 6 inches wide, and 2 inches high.

Those skilled in the art will appreciate the numerous variations that may be made with respect to the present disclosure and which are intended to be captured herein. Other embodiments include all of the various combinations of individual features of each of the embodiments described herein.

All documents cited in the Detailed Description of the Invention are, in relevant part, incorporated herein by reference; the citation of any document is not to be construed as an admission that it is prior art with respect to the present invention.

### Industrial Applicability

The dispensing system described herein advantageously combines features enabling placement of the device under furniture and the like without damaging the furniture or wasting volatile active, while being able to effectively dispense a volatile active in a large room or space.

## Claims

1. A dispensing system (10) for dispensing a material, comprising:
a housing (12) for receipt of a dispenser (38, 38a, 38b) holding a material, wherein the housing (12) includes a sidewall (20) with vents (22), and
a first fan (68a) disposed within the housing,
wherein upon activation, the first fan is adapted to draw air into the housing;
**characterized by**
a second fan (68b),
wherein an impeller of the first fan is coplanar with an impeller of the second fan, and wherein the impeller of the first fan and the impeller of the second fan are adapted to rotate in directions of rotation which are counter-rotational to one another such that the material charged air is diffused from the housing with a 360 degree dispersal pattern.

2. The dispensing system of claim 1, wherein the vents (22) are adapted to draw the air into the housing (12) from a direction different than that of the diffusion of the material.

3. The dispensing system of claim 2, wherein the vents (22) are adapted to draw the air into the housing (12) from an angle greater than about 45 degrees from horizontal.

4. The dispensing system of claim 2, wherein the vents (22) are adapted to draw the air into the housing (12) from a vertical direction.

5. The dispensing system of claim 1, wherein the dispenser (38, 38a, 38b) comprises a blister (50) holding a volatile material and a permeable membrane (56) extending across an open end of the blister.

## Patentansprüche

1. Ein Ausgabesystem (10) zur Ausgabe eines Materials umfasst:
ein Gehäuse (12) zur Aufnahme einer Abgabevorrichtung (38, 38a, 38b), die ein Material enthält, wobei das Gehäuse (12) eine Seitenwand (20) mit Öffnungen (22) und einen ersten Lüfter (68a) umfasst, der in dem Gehäuse angeordnet ist,
wobei der erste Lüfter darauf ausgelegt ist, dass aufgrund der Aktivierung Luft in das Gehäuse eingezogen wird;
**gekennzeichnet durch**
einen zweiten Lüfter (68b),
wobei ein Flügelrad des ersten Lüfters mit dem Flügelrad eines zweiten Lüfters komplanar angeordnet ist, und wobei das Flügelrad des ersten Lüfters und das Flügelrad des zweiten Lüfters so angeordnet sind, dass sie in Richtungen rotieren, die entgegengesetzt zueinander gerichtet sind, sodass die mit dem Material angereicherte Luft mit einem 360° Ausbreitungsmuster aus dem Gehäuse verbreitet wird.

2. Ausgabesystem nach Anspruch 1, wobei die Öffnungen (22) so angeordnet sind, dass Luft aus einer Richtung in das Gehäuse (12) angezogen wird, die sich von der Richtung der Verbreitung des Materials unterscheidet.

3. Ausgabesystem nach Anspruch 2, wobei die Öffnungen (22) so angeordnet sind, dass Luft aus einem Winkel in das Gehäuse (12) angezogen wird, der größer ist als 45°, bezogen auf die Horizontale.

4. Ausgabesystem nach Anspruch 2, wobei die Öffnungen (22) so angeordnet sind, dass Luft aus einer vertikalen Richtung in das Gehäuse (12) angezogen wird.

5. Ausgabesystem nach Anspruch 1, wobei die Ausgabevorrichtung (38, 38a, 38b) einen Blister (50) umfasst, der ein flüchtiges Material und eine permeable Membran (56) enthält, wobei diese sich entlang eines offenen Endes des Blisters erstreckt.

## Revendications

1. Système de distribution (10) pour distribuer une matière, comprenant :
un boîtier (12) pour la réception d'un distributeur (38, 38a, 38b) contenant une matière, dans lequel le boîtier (12) inclut une paroi latérale (20) avec des évents (22), et
un premier ventilateur (68a) disposé à l'intérieur du boîtier,
dans lequel lors d'une activation, le premier ventilateur est adapté pour aspirer de l'air dans le boîtier ;
**caractérisé par**
un second ventilateur (68b),
dans lequel une roue du premier ventilateur est coplanaire avec une roue du second ventilateur, et dans lequel la roue du premier ventilateur et la roue du second ventilateur sont adaptées pour tourner dans des sens de rotation qui sont inverses l'un de l'autre de telle sorte que la matière chargée d'air est diffusée à travers le boîtier avec un motif de dispersion à 360 degrés.

2. Système de distribution selon la revendication 1, dans lequel les évents (22) sont adaptés pour aspirer l'air dans le boîtier (12) à partir d'une direction différente de celle de la diffusion de la matière.

3. Système de distribution selon la revendication 2, dans lequel les évents (22) sont adaptés pour aspirer l'air dans le boîtier (12) à partir d'un angle supérieur à environ 45 degrés par rapport à l'horizontale.

4. Système de distribution selon la revendication 2, dans lequel les évents (22) sont adaptés pour aspirer l'air dans le boîtier (12) à partir d'une direction verticale.

5. Système de distribution selon la revendication 1, dans lequel le distributeur (38, 38a, 38b) comprend une alvéole (50) contenant une matière volatile et une membrane perméable (56) s'étendant à travers une extrémité ouverte de l'alvéole.
